# EUROPÄISCHE PATENTSCHRIFT

(11) **EP 1 627 879 B1**
(45) Veröffentlichungstag und Bekanntmachung des Hinweises auf die Patenterteilung: **18.10.2006**
(21) Anmeldenummer: 04019858.2
(22) Anmeldetag: 20.08.2004
(51) Int. Cl.: C07F 3/00, C07F 19/00, A23K 1/16, A61K 31/28

(54) **Verfahren zur Herstellung von bestimmten Hydrohalogenid-Metallkomplexverbindungen mit spezifischer grobkörniger Struktur**
Process for preparing hydrohalogen metal complexes of specific particle size
Procédé de préparation de complexes métalliques hydrohalogénures de granulométrie spécifique

(43) Veröffentlichungstag der Anmeldung: 22.02.2006
(73) Patentinhaber: Verla-Pharm Arzneimittelfabrik Apotheker H.J. von Ehrlich GmbH & Co. KG, D-82327 Tutzing (DE)
(72) Erfinder: Hopf, Günter, 82327 Tutzing (DE)
(74) Vertreter: Müller-Boré & Partner Patentanwälte

(56) Entgegenhaltungen:
- DE-A- 2 228 101
- DE-A- 3 238 118

## Beschreibung

Die vorliegende Erfindung betrifft ein Verfahren zur Herstellung eines granulatförmigen Hydrohalogenidsalzes einer bestimmten Metallkomplexverbindung, die aus einem zweiwertigen Metallkation als Zentralion und einem Aminodicarbonsäureion und gegebenenfalls Wasser als Liganden aufgebaut ist, wobei das Hydrohalogenidsalz mit spezifischer Teilchengrössenverteilung erhalten wird.

Komplexe aus einem zweiwertigen Metallkation als Zentralion und einem Aminodicarbonsäureliganden sowie deren Hydrohalogenide und unterschiedliche Verfahren zu deren Herstellung sind bekannt. Komplexe aus einem zweiwertigen Metallkation als Zentralion und einem Aminodicarbonsäureliganden, beispielsweise Magnesium-L-aspartat, sind weitgehend problemlos in ihrer Handhabung. Im Gegensatz dazu sind deren Hydrohalogenide, vor allem Magnesium-L-aspartathydrochlorid, meist sehr hygroskopisch, so daß deren Herstellung nur bei extrem niedriger Luftfeuchtigkeit gelingt. Bereits bei einer Luftfeuchtigkeit von mehr als 50% verflüssigt sich Magnesium-L-aspartathydrochlorid, was dessen Weiterverarbeitung extrem erschwert oder sogar unmöglich macht. Eine Weiterverarbeitung zu Tabletten oder Granulaten ist folglich nur in klimatisierten Bereichen mit kontrolliert niedriger Luftfeuchtigkeit möglich. Aufgrund der extremen Hygroskopizität konnte vor allem Magnesium-L-aspartathydrochlorid bislang nur mit organischen Lösungsmitteln granuliert werden, was aus Umweltgründen nicht erwünscht ist.

Um das Problem der Hygroskopizität der vorstehend beschriebenen Hydrohalogenide zu lösen, wurden Versuche unternommen, ein granulatförmiges Produkt mit einer verkleinerten Gesamtoberfläche herzustellen. Alle bislang unternommenen Versuche zur Herstellung eines granulatförmigen Produkts durch Sprühtrocknung lieferten jedoch ein ungenügendes, meist sehr feinpulvriges Material, welches aufgrund der großen Oberfläche oder dem hohen Anteil an sehr feinpulvrigem Material rasch eine honigartige Konsistenz annimmt, was eine Weiterverarbeitung unmöglich macht. Beispielsweise wird ein sehr feinpulvriges Material unter den in DE 32 38 118 A1 (vgl. Beispiele 1 bis 5 von DE 32 38 118 A1) angegebenen Sprühtrocknungsbedingungen (Lufteintrittstemperatur: etwa 180°C; Luftaustrittstemperatür: etwa 120°C) erhalten.

Somit liegt der vorliegenden Erfindung die technische Aufgabe zugrunde, Hydrohalogenide von Komplexverbindungen, die aus einem zweiwertigen Metallkation als Zentralion und einem Aminodicarbonsäureion und gegebenenfalls Wasser als Liganden aufgebaut sind, bereitzustellen, die gute Fließ- und Löseeigenschaften und eine verminderte Hygroskopizität aufweisen sollen.

Diese Aufgabe wird durch Bereitstellen der in den Ansprüchen gekennzeichneten Ausführungsformen gelöst.

Insbesondere wird ein Verfahren zur Herstellung eines granulatförmigen Hydrohalogenids einer Komplexverbindung, die aus einem zweiwertigen Metallkation als Zentralion und einem Aminodicarbonsäureion und gegebenenfalls Wasser als Liganden aufgebaut ist, bereitgestellt, umfassend die Schritte:
(a) Vorlegen des Hydrohalogenids der Komplexverbindung in wäßriger Lösung,
(b) Sprühtrocknen der in Schritt (a) erhaltenen wäßrigen Lösung bei einer Lufteintrittstemperatur von 300 bis 350 °C und einer Luftaustrittstemperatur von 100 bis 140 °C und einem Sprühdruck von 3 bis 5 bar.

Die erfindungsgemäß hergestellten Hydrohalogenide der Komplexverbindungen lassen sich auf unterschiedlichen Wegen herstellen. Beispielsweise können sie durch Vermischen äquimolarer Mengen eines Metallsalzes einer Aminodicarbonsäure (mit einem zweiwertigen Metall, wie beispielsweise Magnesium) und einem entsprechenden Metallhalogenid in wäßriger Lösung hergestellt werden. Es ist jedoch bevorzugt, die Hydrohalogenide der Komplexverbindungen durch Umsetzen einer Aminodicarbonsäure mit einem Hydroxid, Oxid und/oder Carbonat des Metalls (M) in wäßriger Lösung und weiteres Umsetzen der resultierenden wäßrigen Lösung oder Suspension mit einem Halogenid des Metalls (M) und/oder Halogenwasserstoffsäure herzustellen. Durch das letztgenannte Herstellungsverfahren kann von günstigeren und leicht verfügbaren Ausgangsverbindungen ausgegangen werden, wodurch der Gesamtprozeß wirtschaftlicher wird.

Besonders bevorzugt ist es, eine wäßrige Lösung oder Suspension von 2 Mol der jeweiligen Aminodicarbonsäure mit einer wäßrigen Lösung oder Suspension von 1 Mol des entsprechenden Metalloxids, -hydroxids und/oder -carbonats sowie einer wäßrigen Lösung oder Suspension von 1 Mol des entsprechenden Metallhalogenids zu vermischen und solange zu rühren, bis eine klare Lösung erhalten wird. Anstelle des Metallhalogenids kann auch eine äquimolare Menge einer Halogenwasserstoffsäure und eines Metalloxids, -hydroxids und/oder -carbonats verwendet werden. Das Vermischen erfolgt vorzugsweise in einem Temperaturbereich von etwa 20 bis etwa 90°C, oder im Falle einer exothermen Reaktion bei leicht erhöhter Temperatur, bis eine klare Lösung erhalten wird, die durch Filtration gereinigt werden kann. In manchen Fällen kann es vorteilhaft sein, zuerst die Lösung oder Suspension der Aminodicarbonsäure mit dem Metalloxid, -hydroxid oder -carbonat als Feststoff, Lösung oder Suspension zu vermischen und erst danach, wenn eine klare Lösung erhalten worden ist, die Lösung des Metallhalogenids zuzusetzen.

Das Hydrohalogenid der Komplexverbindung ist vorzugsweise eine gemäß der nachfolgenden Formel (I) gekennzeichnete Verbindung: 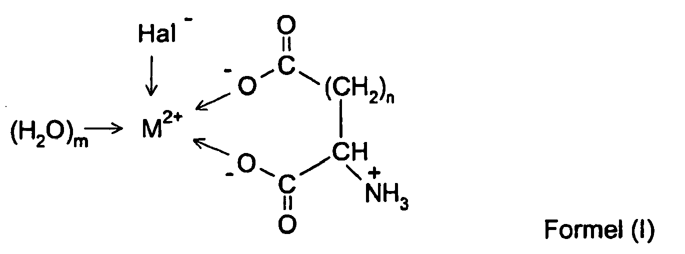 worin M²⁺ ein zweiwertiges Metallkation, Hal⁻ ein Halogenidion, wie Fluorid, Chlorid, Bromid oder Iodid, n 1 oder 2 und m 0 bis 10, vorzugsweise 0, 1, 2 oder 3, sind.

Die verwendbaren Aminodicarbonsäuren unterliegen keinen besonderen Beschränkungen, insoweit sie zur Ausbildung eines Chelats mit einem zweiwertigen Metallkation, wie beispielsweise Magnesium, Calcium oder Eisen, befähigt sind. Ein Fachmann ist in der Lage, eine Vielzahl geeigneter substituierter oder unsubstituierter Aminodicarbonsäuren aufzufinden, die einen stabilen Komplex mit einem zweiwertigen Metallkation bilden können. Besonders bevorzugt ist es, in dem erfindungsgemäßen Verfahren L-Glutaminsäure oder L-Asparginsäure als Aminodicarbonsäure zu verwenden.

Das in dem Hydrohalogenid der Komplexverbindung vorhandene Metall (M), welches das Zentralkation des Komplexes darstellt, kann grundsätzlich jedes zweiwertige Metallkation sein. Vorzugsweise ist das Metall (M) ein Erdalkalimetall, insbesondere Magnesium, Calcium oder Strontium, oder ein Schwermetall, insbesondere Zink, Eisen, Mangan, Cobalt, Kupfer oder Cadmium. In dem erfindungsgemäß erhaltenen Komplex wird das zweiwertige Metallkation als Zentralion von dem zweizähnigen Aminodicarbonsäureliganden komplexiert, wobei sich ein Chelatkomplex ausbildet. Abhängig von Zentralion und Aminodicarbonsäureligand können variierende Mengen an Wasser, üblicherweise bis etwa 10 Moleküle pro Metallkation, gebunden werden. Das Hydrohalogenid entsteht durch Protonierung der Aminogruppe des Aminodicarbonsäureliganden, wobei als Gegenion ein Halogenidion, wie Fluorid, Chlorid, Bromid oder Iodid, gebunden wird.

Gemäß einer bevorzugten Ausführungsform der vorliegenden Erfindung ist in der vorstehenden Formel (I) M Magnesium, Calcium oder Eisen, n 1 oder 2, Hal Chlor und m 0, 1, 2 oder 3. Die Verbindung der Formel (I) ist gemäß einer besonders bevorzugten Ausführungsform ein Erdalkalimetall-L-aspartathydrohalogenid oder ein Erdalkalimetall-L-glutamathydrohalogenid, insbesondere Magnesium-L-aspartat-hydrochlorid oder Magnesium-L-glutamathydrochlorid, oder ein Hydrat davon. Insbesondere bei Verwendung von Magnesium-L-aspartathydrochlorid läßt sich im Rahmen des erfindungsgemäßen Verfahrens ein ausgezeichnet verarbeitbares Produkt mit einer spezifisch grobkörnigen Struktur und einer engen Teilchengrößenverteilung erzeugen.

In dem erfindungsgemäßen Verfahren ist zum Erreichen der vorteilhaften grobkörnigen Struktur des Hydrohalogenids der Komplexverbindung die gezielte Steuerung der Lufteintrittstemperatur und der Luftaustrittstemperatur in dem Sprühtrocknungsschritt (b) in Kombination mit einem geeigneten Sprühdruck wesentlich, um ein Granulat mit einer spezifisch engen Teilchengrößenverteilung, guten Fließ- und Löseeigenschaften und einer verminderten Hygroskopizität zu erhalten. Es wurde überraschenderweise festgestellt, daß bei einer Lufteintrittstemperatur in einem Bereich von 300 bis 350 °C und einer Luftaustrittstemperatur in einem Bereich von 100 bis 140 °C ein stabiles granulatförmiges Produkt mit ausgezeichneter Weiterverarbeitbarkeit erhalten werden kann, wenn die wäßrige Lösung bei einem Sprühdruck in einem Bereich von etwa 3 bis etwa 5 bar eingesprüht bzw. zerstäubt wird. Die genaue Temperatur innerhalb dieser Bereiche hängt von dem der Sprühtrocknung zu unterwerfenden Hydrohalogenid ab. Die genaue Einstellung ist jedoch einem Fachmann innerhalb der vorstehenden Bereiche möglich. Es ist in diesem Zusammenhang ferner überraschend, daß trotz der Verwendung einer vergleichsweise hohen Lufteintrittstemperatur ein grobkörniges Produkt erhalten wird.

Die wäßrige Lösung in Schritt (a) kann von oben oder von unten in einen Sprühtrocknungsturm eingesprüht bzw. zerstäubt werden. Das zur Trocknung verwendete Gas, vorzugsweise Luft, kann im Gleichstrom oder im Gegenstrom zu der versprühten wäßrigen Lösung geführt werden. Es ist jedoch bevorzugt, die eingesprühte wäßrige Lösung, die zu feinen Tröpfchen zerstäubt wird, von oben in einen Sprühtrocknungsturm einzusprühen und das zur Trocknung verwendete Gas dazu im Gleichstrom, d.h. von oben nach unten, zu führen. Anschließend an den Luftaustritt kann ein Zyklon und/oder ein Filter vorgesehen werden, um feines pulverförmiges Material abzutrennen.

Der Sprühdruck in Schritt (b) des erfindungsgemäßen Verfahrens liegt in einem Bereich von etwa 3 bar bis etwa 5 bar. Der Sprühdruck entspricht dem Flüssigkeitsvordruck der Düsen. Die Zerstäubung der in Schritt (a) bereitgestellten Lösung erfolgt üblicherweise durch eine Einstoffdüse bzw. Hohlkegeldüse, die am Düsenaustritt einen hohlkegelartigen Flüssigkeitskonus erzeugt, der in gleichmäßigen Tröpfchen mit einer engen Tröpfchengrößenverteilung resultiert.

Gemäß einer bevorzugten Ausführungsform des erfindungsgemäßen Verfahrens durchlaufen die frisch gesprühten Partikel direkt im Anschluß an den Sprühtrocknungsschritt ein Fließbett, um beispielsweise die Restfeuchte zu verringern. Beim Zusammentreffen der frisch gesprühten Partikel mit den Teilchen im Fließbett bildet sich ein erfindungsgemäßes granulatförmige Produkt mit ausgezeichneten Eigenschaften, das anschließend aus dem Sprühturm, vorzugsweise über einen Überlauf, ausgetragen wird. Ferner kann ein anschließender Siebschritt vorgesehen werden. Besonders bevorzugt ist es, einen Sprühturm mit einem integrierten Fließbett vorzusehen. Die Lufteintrittstemperatur zum Fließbett liegt vorzugsweise in einem Bereich von 110 bis 130 °C, wobei die Temperatur des Produkts im Fließbett vorzugsweise auf etwa 100 bis 125 °C eingestellt wird. Die Höhe des Fließbetts unterliegt grundsätzlich keinen besonderen Beschränkungen, wird jedoch vorzugsweise auf 15 cm bis 30 cm eingestellt. Die Einstellung der Verweilzeit des Produkts in dem Sprühtrockner liegt im handwerklichen Ermessen eines Fachmanns und kann vor allem durch Einstellen der Höhe des Überlaufs und des Materialdurchsatzes bestimmt werden. Der Materialdurchsatz in dem Sprühtrocknungsschritt liegt in einem Bereich von 50 kg bis 200 kg pro Stunde, wobei ein Durchsatz von 70 kg bis 130 kg pro Stunde bevorzugt ist. Das Volumen des Sprühtrockners unterliegt keinen besonderen Beschränkungen. Es liegt jedoch vorzugsweise in einem Bereich von etwa 5 bis 20 m³, wobei ein Volumen von etwa 8 m³ aus wirtschaftlichen Gründen am häufigsten angewendet wird.

Im Rahmen des erfindungsgemäßen Verfahrens zur Herstellung des granulatförmigen Hydrohalogenids der hier betreffenden Komplexverbindung läßt sich ein Produkt mit einem sehr günstigen Schüttvolumen erreichen. Vorzugsweise liegt das Schüttvolumen [Volumen des unkomprimierten Produkts (ml) / 100 g des Produkts] in einem Bereich von 150 bis 180 ml/100g, wobei ein Schüttvolumen von etwa 170 ml/100g besonders bevorzugt ist.

Die Konzentration der wäßrigen Lösung in Schritt (a) unterliegt grundsätzlich keinen besonderen Beschränkungen. Es ist jedoch bevorzugt, die Konzentration der wäßrigen Lösung in Schritt (a) auf 0,5 bis 3 mol/l, vorzugsweise 1 mol/l bis 2 mol/l, besonders bevorzugt auf etwa 1,3 mol/l bis 1,5 mol/l einzustellen.

Ein weiterer Gegenstand der vorliegenden Erfindung betrifft das Granulat eines Hydrohalogenids einer Komplexverbindung, die aus einem zweiwertigen Metallkation als Zentralion und einem Aminodicarbonsäureion und gegebenenfalls Wasser als Liganden aufgebaut ist, wobei ≤ 10 % der Teilchen eine Teilchengröße von < 50 µm und ≤ 10% der Teilchen eine Teilchengröße von > 400 µm aufweisen, erhalten nach dem vorstehend beschriebenen erfindungsgemäßen Verfahren.

Besonders bevorzugt weisen ≥ 80 % der Teilchen, vorzugsweise ≥ 85 % der Teilchen, des Granulats eine Teilchengröße in einem Bereich von etwa 100 µm bis etwa 315 µm auf. Ferner ist es besonders bevorzugt, daß ≥ 50 % der Teilchen, vorzugsweise ≥ 55 % der Teilchen, des Granulats eine Teilchengrösse in einem Bereich von etwa 140 µm bis etwa 250 µm aufweisen. Wie bereits vorstehend ausgeführt, liegt das Schüttvolumen [Volumen des unkomprimierten Produkts (ml) / 100 g des Produkts] eines solchen Granulats vorzugsweise in einem Bereich von 150 bis 180 ml/100g, wobei ein Schüttvolumen von etwa 170 ml/100g besonders bevorzugt ist

Das nach dem erfindungsgemäßen Verfahren hergestellte Granulat eines Hydrohalogenids einer Magnesium-enthaltenden Komplexverbindung eignet sich zur verwendung in der Magnesiumtherapie und als Tierfutterzusatz. Die erfindungsgemäß erhaltenen Granulate solcher Hydrohalogenide der hier betreffenden Magnesiumkomplexverbindungen sind wertvolle Pharmazeutika und Zusätze zu Tierfutter. So wird beispielsweise Magnesium-L-aspartathydrochlorid zur gezielten Magnesiumtherapie und auch als Tierfutterzusatz und auch als mineralisches Ergänzungsfutter von Nutztieren eingesetzt. Die Verbindungen können in fester granulärer Form oder in wäßriger Lösung eingesetzt werden.

Die nachfolgenden Beispiele werden angegeben, um die Erfindung näher zu erläutern, ohne diese dadurch zu beschränken.

### Beispiele

### Beispiel 1

### Herstellung von Magnesium-L-aspartathydrochlorid

1753 I entmineralisiertes Wasser werden vorgelegt und unter Rühren mit 836 kg L-Asparginsäure versetzt. Zu der resultierenden Dispersion werden 130 kg Magnesiumoxid als Pulver zugesetzt und das Gemisch wird unter Rühren auf 60 °C erwärmt. Anschließend werden unter Rühren 628 kg Magnesiumchlorid (MgCl_{2•}6H₂O) zugesetzt und das Gemisch wird unter Rühren für 2 h auf 60 °C erwärmt. Die Lösung wird anschließend bei 60 °C filtriert und bei einer Lufteintrittstemperatur von etwa 320°C und einer Luftaustrittstemperatur von etwa 120°C bei einem Sprühdruck von etwa 4 bar in einem Sprühtrockner der Firma Niro sprühgetrocknet.

Als Endprodukt wird ein Magnesium-L-aspartathydrochlorid mit der folgenden Teilchengrössenverteilung erhalten: < 100µm (6,19%), 100-140µm (19,48%), 140-250µm (57,84%), 250-315µm (10,81%), 315-400µm (4,78%) und 400-500µm (0,90%). Das Schüttvolumen dieses Endprodukts betrug 170ml/100g.

### Beispiel 2

### Herstellung von Magnesium-L-glutamathydrochlorid

936 l entmineralisiertes Wasser werden auf 60°C erwärmt, worauf 484 kg L-Glutaminsäure unter Rühren zugesetzt werden. Zu der erhaltenen Dispersion werden 67 kg Magnesiumoxid als Pulver portionsweise unter ständigem Rühren zugesetzt. Die Temperatur steigt an und die Lösung wird nach etwa 1 h klar. Zu dieser Lösung wird eine Lösung aus 480 kg Magnesiumbromidhexahydrat und 384 I Wasser (35 %ige Lösung) zugesetzt. Unter Verwendung von Wasser wird eine 30 %ige Konzentration des Komplexes eingestellt. Die Lösung wird filtriert und anschließend unter den in Beispiel 1 beschriebenen Bedingungen sprühgetrocknet.

Dabei wird Magnesium-L-glutamathydrochlorid als weißes Pulver in 100 %iger Ausbeute erhalten.

### Beispiel 3

### Herstellung von Calcium-L-aspartathydrochlorid

1010 l entmineralisiertes Wasser werden auf 60°C erwärmt, worauf unter Rühren 509 kg L-Asparginsäure zugesetzt werden. Zu der erhaltenen Dispersion werden 142 kg Calciumhydroxid in Form eines Pulvers portionsweise unter ständigem Rühren zugesetzt. Die Temperatur steigt weiter an und die Lösung wird nach etwa 1 h klar. Zu dieser Lösung wir eine Lösung von 281 kg Calciumchlorid-Dihydrat in 325 l Wasser (35 %ige Lösung) zugegeben. Mit Wasser wird die Konzentration, bezogen auf den Komplex, auf 30 % eingestellt. Anschließend wird filtriert und nach der in Beispiel 1 beschriebenen Weise sprühgetrocknet.

Dabei wird Calcium-L-aspartathydrochlorid in Form eines weißen Pulvers in 100%iger Ausbeute erhalten.

### Beispiel 4

### Herstellung von Zink-L-aspartathydrochlorid

1025 l entmineralisiertes Wasser werden auf 60°C erwärmt, worauf unter Rühren 464 kg L-Asparginsäure zugesetzt werden. Der erhaltenen Dispersion werden 142 kg Zinkoxid in Form eines Pulvers portionsweise unter ständigem Rühren zugesetzt.

Die Temperatur steigt etwas an, die Lösung wird jedoch nicht klar. Deshalb wird die Temperatur auf etwa 90°C erhöht, worauf eine klare Lösung erhalten wird. Durch Zugabe von Wasser wird die Konzentration auf 30 % eingestellt.

Der erhaltenen Lösung wird eine Lösung von 238 kg Zinkchlorid in 441 l Wasser (35 %ige Lösung) zugesetzt. Die Konzentration, bezogen auf den Komplex, wird mit Wasser auf 30 % eingestellt. Dann wird filtriert und nach der in Beispiel 1 beschriebenen Weise sprühgetrocknet. Dabei wird Zink-L-aspartathydrochlorid in Form eines weißen Pulvers in 100 %iger Ausbeute erhalten.

### Beispiel 5

### Herstellung von Magnesium-L-aspartathydrochlorid

541 kg L-Asparaginsäure werden in 1016 I entmineralisiertem Wasser unter Rühren dispergiert, wobei auf 60°C erwärmt wird. Zu dieser Dispersion werden 592 kg einer 25 Gew.-%igen Salzsäure und anschließend 164 kg Magnesiumoxid als Pulver zugesetzt und gerührt. Nachdem eine klare Lösung erhalten worden ist, wird diese in der in Beispiel 1 beschriebenen Weise filtriert und sprühgetrocknet, wobei Magnesium-L-aspartathydrochlorid in Form eines weißen Pulvers in 100%iger Ausbeute erhalten wird.

## Patentansprüche

1. Verfahren zur Herstellung eines granulatförmigen Hydrohalogenids einer Komplexverbindung, die aus einem zweiwertigen Metallkation als Zentralion und einem Aminodicarbonsäureion und gegebenenfalls Wasser als Liganden aufgebaut ist, umfassend die Schritte:
(a) Vorlegen des Hydrohalogenids der Komplexverbindung in wäßriger Lösung,
(b) Sprühtrocknen der in Schritt (a) erhaltenen wäßrigen Lösung bei einer Lufteintrittstemperatur von 300 bis 350 °C und einer Luftaustrittstemperatur von 100 bis 140 °C und einem Sprühdruck von 3 bis 5 bar.

2. Verfahren nach Anspruch 1, wobei die frisch gesprühten Partikel nach Schritt (b) zur Verringerung der Restfeuchte durch ein Fließbett geführt werden und gegebenenfalls anschließend einem Siebschritt unterworfen werden.

3. Verfahren nach Anspruch 1 oder 2, wobei das Hydrohalogenid der Komplexverbindung durch Umsetzen einer Aminodicarbonsäure mit einem Hydroxid, Oxid und/oder Carbonat des Metalls (M) in wäßriger Lösung und weiteres Umsetzen der resultierenden wäßrigen Lösung oder Suspension mit einem Halogenid des Metalls (M) und/oder Halogenwasserstoffsäure hergestellt wird.

4. Verfahren nach einem der Ansprüche 1 bis 3, wobei das Hydrohalogenid der Komplexverbindung eine in der nachfolgenden Formel (I) gezeigte Verbindung ist: 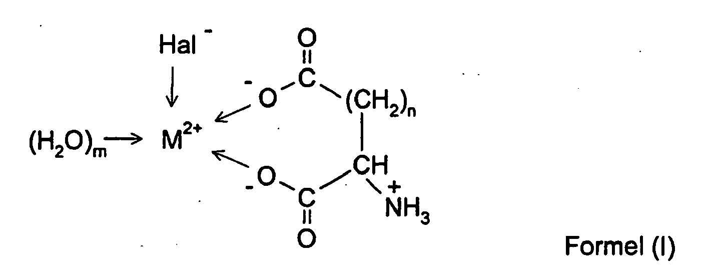 worin M²⁺ ein zweiwertiges Metallkation, Hal⁻ ein Halogenidion, n 1 oder 2 und m 0 bis 10 sind.

5. Verfahren nach einem der Ansprüche 1 bis 4, wobei die Aminodicarbonsäure L-Glutaminsäure oder L-Asparginsäure ist.

6. Verfahren nach einem der Ansprüche 1 bis 5, wobei das zweiwertige Metall (M) ein Erdalkalimetall oder ein Schwermetall ist.

7. Verfahren nach einem der Ansprüche 1 bis 6, wobei das Hydrohalogenid der Komplexverbindung ein Erdalkalimetall-L-aspartathydrohalogenid oder ein Erdalkalimetall-L-glutamathydrohalogenid ist.

8. Verfahren nach einem der Ansprüche 1 bis 7, wobei das Hydrohalogenid der Komplexverbindung Magnesium-L-aspartat-hydrochlorid oder Magnesium-L-glutamathydrochlorid ist.

9. Verfahren nach einem der Ansprüche 1 bis 8, wobei die Konzentration der wäßrigen Lösung in Schritt (a) auf 0,5 mol/l bis 3 mol/l eingestellt wird.

10. Granulat eines Hydrohalogenids einer Komplexverbindung, die aus einem zweiwertigen Metallkation als Zentralion und einem Aminodicarbonsäureion und gegebenenfalls Wasser als Liganden aufgebaut ist, wobei ≤ 10 % der Teilchen eine Teilchengröße von < 50 µm und ≤ 10% der Teilchen eine Teilchengröße von > 400µm aufweisen, erhalten nach dem in einem der AnSprüche 1 bis 9 definierten Verfahren.

11. Granulat nach Anspruch 10, wobei ≥ 80 % der Teilchen eine Teilchengröße in einem Bereich von etwa 100 µm bis etwa 315 µm aufweisen.

12. Granulatförmiges Hydrohalogenid nach Anspruch 10 oder 11, wobei ≥ 50 % der Teilchen eine Teilchengröße in einem Bereich von etwa 140 µm bis etwa 250 µm aufweisen.

## Claims

1. Process for preparing a granular hydrohalide of a complex compound which is composed of a divalent metal cation as central ion and of an amino dicarboxylic acid ion and, where appropriate, water as ligand comprising the steps:
(a) preparation of an aqueous solution of the hydrohalide of the complex compound,
(b) spray drying of the aqueous solution obtained in step (a) at an air inlet temperature of from 300 to 350°C and at an air outlet temperature of from 100 to 140°C and with a spraying pressure of from 3 to 5 bar.

2. Process according to Claim 1, where the freshly sprayed particles after step (b) are passed through a fluidized bed to reduce the residual moisture, and are subjected where appropriate subsequently to a sieving step.

3. Process according to Claim 1 or 2, where the hydrohalide of the complex compound is prepared by reacting an amino dicarboxylic acid with a hydroxide, oxide and/or carbonate of the metal (M) in aqueous solution and further reaction of the resulting aqueous solution or suspension with a halide of the metal (M) and/or hydrohalic acid,

4. Process according to any of Claims 1 to 3, where the hydrohalide of the complex compound is a compound shown in formula (I) below: 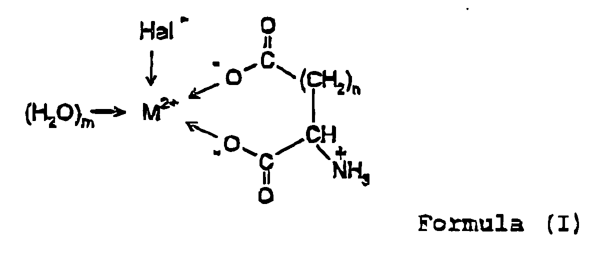 in which M²⁺ is a divalent metal cation, Hal⁻ is a halide ion, n is 1 or 2 and m is 0 to 10.

5. Process according to any of Claims 1 to 4, where the amino dicarboxylic acid is L-glutamic acid or L-aspartic acid.

6. Process according to any of Claims 1 to 5, where the divalent metal (M) is an alkaline earth metal or a heavy metal.

7. Process according to any of Claims 1 to 5, where the hydrohalide of the complex compound is an alkaline earth metal L-aspartate hydrohalide or an alkaline earth metal L-glutamate hydrohalide.

8. Process according to any of Claims 1 to 7, where the hydrohalide of the complex compound is magnesium L-aspartate hydrochloride or magnesium L-glutamte hydrochloride.

9. Process according to any of Claims 1 to 8, where the concentration of the aqueous solution in step (a) is set at from 0.5 mol/l to 3 mol/l.

10. Granules of a hydrohalide of a complex compound which is composed of a divalent metal cation as ≤ 10% of the particles have a particle size of < 50 µm and ≤ 10% of the particles have a particle size of > 400 µm, obtained by the process defined in any of Claims 1 to 9.

11. Granules according to Claim 10, where ≥ 80% of the particles have a particle size in a range from about 100 µm to about 315 µm.

12. Granular hydrohalide according to Claim 10 or 11, where ≥ 50% of the particles have a particle size in a range from about 140 µm to about 250 µm.

## Revendications

1. Procédé de préparation d'un hydrohalogénure granulaire d'un composé complexe qui est constitué d'un cation métallique bivalent comme ion central et d'un ion d'acide amino-dicarboxylique et éventuellement d'eau à titre de ligands, comprenant les étapes consistant à :
(a) placer l'hydrohalogénure du composé complexe dans une solution aqueuse,
(b) sécher par pulvérisation la solution aqueuse obtenue à l'étape (a) à une température d'entrée d'air de 300 à 350° C et à une température de sortie d'air de 100 à 140° C et à une pression de pulvérisation de 3 à 5 bar.

2. Procédé selon la revendication 1, les particules récemment pulvérisées selon l'étape (b) étant acheminées par un lit fluidisé pour réduire l'humidité résiduelle et étant ensuite soumises éventuellement à une étape de tamisage.

3. Procédé selon la revendication 1 ou 2, l'hydrohalogénure du composé complexe étant préparé en mélangeant un acide amino-dicarboxylique avec un hydroxyde, un oxyde et/ou un carbonate du métal (M) dans une solution aqueuse et en mélangeant ensuite la solution aqueuse ou la suspension obtenue avec un halogénure du métal (M) et/ou de l'hydracide halogéné.

4. Procédé selon l'une quelconque des revendications 1 à 3, l'hydrohalogénure du composé complexe étant un composé représenté dans la formule (I) suivante : 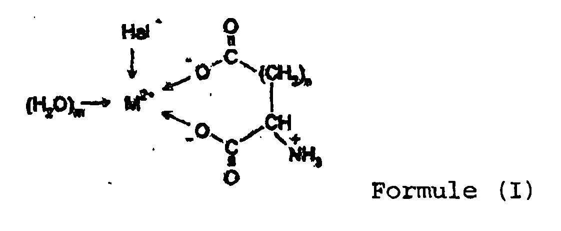 dans laquelle M²⁺ est un cation métallique bivalent, Hal⁻ est un ion halogène, n vaut 1 ou 2 et m vaut 0 à 10.

5. Procédé selon l'une quelconque des revendications 1 à 4, l'acide amino-dicarboxylique étant l'acide L-glutamique ou l'acide L-aspartique.

6. Procédé selon l'une quelconque des revendications 1 à 5, le métal bivalent (M) étant un métal alcalino-terreux ou un métal lourd.

7. Procédé selon l'une quelconque des revendications 1 à 6, l'hydrohalogénure du composé complexe étant un L-aspartate-hydrohalogénure de métal alcalino-terreux ou un L-glutamate-hydrohalogénure de métal alcalino-terreux.

8. Procédé selon l'une quelconque des revendications 1 à 7, l'hydrohalogénure du composé complexe étant le chlorhydrate de magnésium - L-aspartate ou le chlorhydrate de magnésium - L-glutamate.

9. Procédé selon l'une quelconque des revendications 1 à 8, la concentration de solution aqueuse à l'étape (a) étant ajustée de 0,5 moles/l à 3 moles/l.

10. Granulat d'un hydrohalogénure d'un composé complexe qui est constitué d'un cation métallique bivalent comme ion central et d'un ion d'acide amino-dicarboxylique et éventuellement d'eau à titre de ligands, ≤ 10 % des particules présentant une taille de particule < 50 µm et ≤ 10 % des particules présentant une taille de particule > 400 µm, obtenues selon le procédé défini dans l'une quelconque des revendications 1 à 9.

11. Granulat selon la revendication 10, ≥ 80 % des particules présentant une taille de particule située dans une plage d'environ 100 µm à environ 315 µm.

12. Hydrohalogénure granulaire selon la revendication 10 ou 11, ≥ 50 % des particules présentant une taille de particule située dans une plage d'environ 140 µm à environ 250 µm.
